# EUROPEAN PATENT SPECIFICATION

(11) **EP 1 588 710 B1**
(45) Date of publication and mention of the grant of the patent: **02.09.2009**
(21) Application number: 05006885.7
(22) Date of filing: 30.03.2005
(51) Int. Cl.: A61K 36/63

(54) **Anti-allergic composition and method for denaturing allergens**
Antiallergische Zusammensetzung sowie Verfahren zur Denaturierung von Allergenen
Composition anti-allergique et méthode pour la dénaturalisation d'allergènes

(30) Priority: 06.04.2004 JP 2004111774
(43) Date of publication of application: 26.10.2005
(73) Proprietor: Sumika Enviro-Science Co., Ltd., Nishinomiya-shi Hyogo (JP)
(72) Inventor: Shigemura, Takahiro, Kobe-shi, Hyogo (JP)
(74) Representative: Vossius & Partner

(56) References cited:
- EP-A- 1 157 701
- WO-A-96/14064
- GB-A- 835 842
- US-A1- 2003 170 331
- DATABASE WPI Section Ch, Week 198910 Derwent Publications Ltd., London, GB; Class C03, AN 1989-073875 XP002335141 & JP 01 027466 A (MATSUSHITA ELECTRIC WORKS LTD) 30 January 1989 (1989-01-30)

## Description

This invention relates to an anti-allergic composition for denaturing the allergen originated mainly from house dust mites, hair of pets and various kinds of pollens, and a filter or sheet treated with the anti-allergic composition. In addition, it relates to a method for denaturing allergens by using the anti-allergic composition.

A lot of people have suffered from allergic diseases like asthma, atopic dermatitis and so on for long years. Mainly house dust mites that live in the house, hair of pets and various kinds of pollens can be the substances that cause these allergic diseases. Recently, medical treatments that use drugs are applied to allergic patients. On the other hands, removing allergens that cause allergic disease from the environment where allergic patients live is also a useful method that prevents from the exposure to the allergens. It is reported in Japan, Europe and the United States of America that removing allergens can improve the condition of patients.

An air cleaner is used to catch allergens of house dust, dust mites and pollens in the environment. To increase the efficiency, the method of using a fine filter and gathering them by electricity have been proposed. However, there are problems of increasing pressure loss and electricity cost. Further, the allergens caught by the filter keep their activity, so there is the danger of suffering from allergens when throwing the allergens away from the filter. Furthermore, some methods for denaturing allergens on the filter have been proposed. As the method for denaturing allergens, methods of heating, irradiating ultraviolet rays, disposing physically and holding the materials for absorbing allergens have been reported. For example, JP Hei06-154298A discloses a method of using ultraviolet rays and JP 2000-5531A discloses filters treated with an extract of tea leaf; however, these physical methods have some problems; namely, machines are complex and large, and energy cost is high. Furthermore, JP 2002-326944A discloses filters treated with tannic acid and hygroscopic materials; however, the filters treated with tannic acid cause coloring when tap water wets them, because the tannic acid tends to react with iron.

Various kinds of the agents for denaturing allergens have been recently proposed. For example, tannic acid (USP 4,806,526), extract of tea leaf, hydroxyapatite, epicatechin, epigallocatechin, epicatechingallate, gallic acid and gallic acid ester with C₁ to C₄ alcohol (JP Hei06-279273A) and extract of some Oleaceae plants (JP 2003-055122A) are proposed. However, these products are formulated as a solution and sprayed in the environment, and moisture is effective for denaturing allergens. Therefore, the filters that are treated with such products cannot practically denature allergens without moisture. Even if the filters are wetted by a sprayer, it is hard to keep the efficacy for denaturing allergens because of the gradual evaporation of the water.

In addition, acaricides are used for controlling house dust mites. However, the house dust mites, such as *Dermatophagoides farinae*, *Dermatophagoides pteronyssinus*, and so on can be the source of allergens even after dying and these dead bodies of the house dust mites gradually decompose and release fine particles of allergens. As a result, the treatment with acaricides is insufficient to denature allergens.

This invention is useful for denaturing allergens in the environment.

This invention provides a composition comprising at least one plant or extract selected from the Oleaceae group consisting of *Olea spp.* and *Ligustrum spp.,* and a hygroscopic material in a specific percentage. The composition shows excellent denaturing efficacy of allergens, even after drying.

Further, this invention provides a method for denaturing allergens existing in an environment by applying the anti-allergic composition to a filter. Furthermore, this invention also provides a filter or sheet treated with the anti-allergic composition.

An anti-allergic composition of the present invention comprises at least one plant or extract from a plant selected from the Oleaceae group consisting of *Olea spp*. and *Ligustrum spp*., and, a hygroscopic material.

Examples of the Oleaceae group include *Olea europaea*, *Ligustrum obtysifoliim*, *L. tschonoskii*, *L*. *ovalifolium, L. hisauchii*, *L. ibota*, *L. japonicum* and *L*. *lucidum*. As the above plant, which may be present in the composition of the invention, parts of the plant may be used. For example, fresh leaves, fruits or bark can be used as well as dry ones for the anti-allergic composition. The extract can also be extracted from leaves or fruits with water or organic solvents. Examples of the solvent include hydrophilic solvents such as water, methanol, ethanol, isopropyl alcohol, benzyl alcohol, acetic acid, acetone, dimethyl formamide, dimethyl acetamide, dimethyl sulfoxide, ethylene glycol, propylene glycol, diethylene glycol, triethylene glycol, dipropylene glycol, hexylene glycol, polyethylene glycol, glycerin, ethylene glycol monomethyl ether, ethylene glycol monoethyl ether, diethylene glycol monomethyl ether, diethylene glycol monoetyl ether, diethylene glycol monobutyl ether, diethylene glycol monoethyl ether acetate, gamma-butyrolactone and sulfolane; and hydrophobic solvents such as dimethylnaphthalene, dodecylbenzene, liquid paraffin, isophorone, kerosene, dibutyl adipate, diethyl phthalate, diethylene glycol monobutyl ether acetate, propylene carbonate, palm oil, rapeseed oil, cottonseed oil, castor oil and soy bean oil.

The extracts can be used as they are, and the addition of an enzyme such as beta-glucosidase may enhance allergen denaturing activity.

Examples of the hygroscopic material include alkaline earth metal salts such as chlorides and sulfates of magnesium and calcium. Calcium chloride and magnesium chloride are preferably used. Further, calcium chloride is more preferably used, because calcium salts also possess anti-allergic activity.

Oleuropein, which can be isolated from olives and the leaves and bark of the olive tree, can be used in place of the Oleaceae plant or extract in the present invention.

The content of Oleaceae plant or extract in the anti-allergic composition is generally from 10 to 90% by weight. In case that the concentrated extract or oleuropein is used, the content is generally from 0.01 to 50% by weight, preferably from 0.1 to 20% by weight. The content of the hygroscopic material is from 0.5 to 25% by weight.

The composition of the present invention is preferably liquid, because it is easily applied to the environment. In case of making the filter or the sheet of the present invention, various types of the composition of the present invention including liquid, paste and powder can be used and can be applied to the filter or sheet.
Liquid type is most preferable for this process. Further, an addition of an aqueous resin is effective for preventing dust. Examples of the aqueous resin include polyvinyl alcohol, polyacrylic acid, polyethylene glycol, polyacrylic acid salt and polyvinylpyrrolidone.

The material for the filter or sheet of the present invention is not restricted. Examples of the material include polyethylene, polypropylene, polyester, polyamide and polycarbonate. The filter or sheet may be a non-woven net or molten net. Furthermore, natural fibers such as paper, cotton, hemp and silk can be used. Inorganic materials such as glass fiber can also be used. These materials can be suitably selected for the purpose. The filter can be a filter for an air cleaner or air conditioner, and the sheet can be used for mask, dust bag, dust cleaning wiper and so on.

When the anti-allergic composition of the present invention is used for denaturing a dust mite allergen, a combination with use of an acaricide having lethal activity and/or repellent activity against house dust mites is effective. Examples of the acaricide include benzyl alcohol, benzyl benzoate, phenyl salicylate, cinnamaldehyde, hyssop oil, carrot seed oil, pyrethroid compounds such as natural pyrethrins, phenothrin and permethrin, organophosphorus compounds such as fenitrothion, malathion, fenthion and diazinon, dicofol, chlorobenzilate, hexythiazox, tebufenpyrad and pyridaben.

In addition, the anti-allergic composition of the present invention can comprise a fungicide or bactericide for preventing the attack by fungi and bacteria. It may be effective because the anti-allergic composition containing a hygroscopic material may be attacked by fungi and bacteria. Examples of the fungicide and bactericide include 5-chloro-N-methylisothiazolone, methylenebisthiocyanate, 2-bromo-2-nitropropan-1,3-diol, glutaraldehyde, iodopropynylbutylcarbamate, zinc salt of pyridinethiol-N-oxide, 1,2-benzoisothiazolone, 1,2-dibromo-2,4-dicyanobutane, chlorhexydine gluconate, 2-isopropyl-5-methylphenol, 3-methyl-4-isopropylphenol, o-phenylphenol, methyl p-hydroxybenzoate, ethyl p-hydroxybenzoate, propyl p-hydroxybenzoate, butyl p-hydroxybenzoate, p-chloro-m-xylenol, p-chloro-m-cresol, polylysine, benzalkonium chloride, didecyldimethylammonium chloride, cethylpyridinium chloride, N-n-butylbenzoisothiazolone, N-octylisothiazolone, 2-(4-thiazolyl)benzimidazole, methyl 2-benzimidazolecarbamate, tetrachloroisophthalonitrile, diiodomethyl p-tolyl sulfone, p-chlorophenyl-3-iodopropargylformal, 2,3,5,6-tetrachloro-4-(methylsulfonyl)pyridine, glycerin ester of fatter acid and hinokitiol.

The anti-allergic composition of the present invention can further comprise solvents, surfactants, chelating agents, anticorrosive agents, binders, thickeners, perfumes, anti-scale agents, antifoaming agents, antistatic agents, softeners and so on, if necessary.

The anti-allergic composition of the present invention can be used for applying it to an environment as well as for producing the anti-allergic filter or sheet as described above. The objects of the application are typically ones contacted directly with humans and their environment, such as storing space and living space. Examples of the object include carpet, tatami mat, floor, floor cover, bed, bedclothes, sofa, stuffed toy, clothes, curtain, cabinet and living space.

The anti-allergic composition of the present invention is effective for denaturing allergens such as house dust mites, hair and skin of pets, cockroaches, feathers, fungi and plants, especially house dust mites. Typical house dust mites are Cheyletidae and Acaridae, of which most important species causing allergy are *Dermatophagoides farinae* and *Dermatophagoides pteronyssinus*. The house dust mites cause allergy by not only living mites but also their dead bodies and excreta. Fungi germinate in the humid condition and become allergens if human inhales fungi. As plant allergens, cedar, Japanese cypress, hog weed, timothy, Zelkova tree, mugwort and vernal grass are exemplified.

### EXAMPLES

The present invention is described in the following examples and experiments in detail.

### Examples 1-6 and Reference examples 1-3

Each component shown in Tables 1 and 2 was mixed, agitated thoroughly and then each uniform liquid formulation was obtained. Percent in the Tables means percent by weight.

**Table 1**

| Examples | 1 | 2 | 3 | 4 | 5 | 6 |
|---|---|---|---|---|---|---|
| Extract of *Ligustrum* leaves | 96.9 | | | | | |
| Extract of *Olea* dried leaves | | 96.9 | | 82.9 | 82.9 | |
| Extract of *Olea* leaves | | | 96.9 | | | |
| Oleuropein | | | | | | 0.1 |
| Beta-glucosidase | 0.1 | 0.1 | 0.1 | 0.1 | 0.1 | 0.1 |
| Calcium chloride dihydrate | 2 | 2 | 2 | 2 | 2 | 2 |
| Ethanol | | | | 15 | 13 | 20 |
| Polyvinylpyrrolidone | 1 | 1 | 1 | | | |
| Benzyl alcohol | | | | | 2 | |
| Distilled water | | | | | | 77.8 |

**Table 2**

| Reference examples | 1 | 2 | 3 |
|---|---|---|---|
| Oleuropein | 0.1 | | |
| Beta-glucosidase | 0.1 | | 0.1 |
| Extract of *Olea* leaves | | | 98.9 |
| Calcium chloride dihydrate | | 2 | |
| Polyvinylpyrrolidone | | 1 | 1 |
| Ethanol | 20 | | |
| Distilled water | 79.8 | 97 | |

### Experiment 1: Measurement of denaturing efficacy for dust mite allergen

Two grams of each of Examples 1 to 6 and Reference examples 1 to 3 were sprayed on a piece of polyester felt (6.6 cm in diameter) by using a trigger type sprayer, and then dried at room temperature for one night to give a filter. The filter was set in the hose of vacuum cleaner, and 0.05g of standard house dust (containing approximately 1000ng/g of mite allergen) was collected on the filter. After one day, the filter was put into a polyethylene bag, and mite allergen on the felt was extracted with 10 ml of phosphate buffered saline (pH 7.0 containing 15% of Bovine Serum Albumin) by crumpling for one minute. The extracted solution was centrifuged for 60 minutes with 12,000 rpm, and the amount of mite allergen in the supernatant was estimated by the simple detecting kit for the house mite allergen, MITEY CHECKER (product of SHINTO FINE Co., Ltd.). The standard for intensity of the color change for detecting the mite allergen was listed in Table 3. Blank means the same procedure except for spraying the anti-allergic composition. The results are shown in Table 4.

**Table 3**

| Score | Intensity of the color change | Amount of mite allergen |
|---|---|---|
| ++ | Thick, apparent line | > 35 micro grams (> 350 mites) |
| + | Apparent line | 10 micro grams (100 mites) |
| +- | Slight color change | 5 micro grams (50 mites) |
| - | No color change | < 1 micro gram (<10 mites) |

**Table 4**

| | Score of Mitey Checker |
|---|---|
| Blank | ++ |
| Example 1 | - |
| Example 2 | - |
| Example 3 | - |
| Example 4 | - |
| Example 5 | - |
| Example 6 | - |
| Reference example 1 | ++ |
| Reference example 2 | + |
| Reference example 3 | ++ |

### Experiment 2: Measurement of denaturing efficacy of anti-allergen filter

The filter of air conditioner made of plastics (Daikin Co., Ltd.) was divided into three pieces. One piece is not treated, each of the compositions of Example 6 and Reference example 1 was sprayed to the other two pieces, respectively, and they were dried. The air conditioner as it was operated about 3 hours/day for 7 days. The dusts of each piece of the filter were absorbed by a vacuum cleaner with DUST SAMPLER (product of SHINTO FINE Co., Ltd.) and MITEY FELT (product of SHINTO FINE Co., Ltd.). Their felts were put into a polyethylene bag and mite allergen of the felt was extracted with 10 ml of phosphate buffered saline (pH 7.0 containing 15% of Bovine Serum Albumin) by crumpling for one minute. The extracted solution was centrifuged for 60 minutes with 12,000 rpm, and the amount of mite allergen in the supernatant was estimated by the same method as experiment 1. The results are shown in Table 5.

**Table 5**

| | Score of Mitey Checker |
|---|---|
| Blank | + |
| Example 6 | - |
| Reference example 1 | + |

## Claims

1. An anti-auergic composition which comprises at least one plant or extract selected from the Oleaceae group consisting of *Olea spp*. and *Ligustrum spp*., and a hygroscopic material wherein the amount of the hygroscopic material is 0.5 to 25% by weight of the composition.

2. An anti-allergic composition according to claim 1, which comprises an extract of *Olea spp*.

3. An anti-allergic composition which comprises oleuropein and a hygroscopic material.

4. An ant-allergic composition according to any of claims 1 to 3, wherein the hygroscopic material is an alkaline earth metal salt.

5. A filter or sheet treated with the anti-allergic composition described in any one of claims 1 to 4.

6. A method for denaturing allergens existing in an environment by applying the anti-allergic composition described in any one of claims 1 to 4 to a filter.

7. Use of an anti-allergic composition which comprises at least one plant or extract selected from the Oleaceae group consisting of *Olea spp*. and *Ligustrum spp*., and a hygroscopic material for denaturing allergens.

8. Use of an anti-allergic composition which comprises oleuropein and a hygroscopic material for denaturing allergens.

9. Use of the anti-allergic composition for denaturing allergens according to claim 7 or 8, wherein the hygroscopic material is an alkaline earth metal salt.

10. The anti-allergic composition according to claim 4, wherein the hygroscopic material is a chloride or sulphate of magnesium or calcium,

11. The anti-allergic composition according to claim 4, wherein the hygroscopic material is calcium chloride or magnesium chloride.

## Patentansprüche

1. Antiallergische Zusammensetzung umfassend mindestens eine Pflanze oder einen Extrakt, ausgewählt aus der Oleaceae-Gruppe bestehend aus *Olea spp*. und *Ligustrum spp*., und ein hygroskopisches Material, wobei die Menge an hygroskopischem Material 0,5 bis 25 Gew.-% der Zusammensetzung beträgt.

2. Antiallergische Zusammensetzung gemäß Anspruch 1, umfassend einen Extrakt aus *Olea spp*.

3. Antiallergische Zusammensetzung umfassend Oleuropein und ein hygroskopisches Material.

4. Antiallergische Zusammensetzung gemäß einem der Ansprüche 1 bis 3, wobei das hygroskopische Material ein Erdalkalimetallsalz ist.

5. Filter oder Flächengebilde, behandelt mit der antiallergischen Zusammensetzung gemäß einem der Ansprüche 1 bis 4.

6. Verfahren zur Denaturierung von Allergenen, die in einer Umgebung vorhanden sind, durch Anwenden der antiallergischen Zusammensetzung gemäß einem der Ansprüche 1 bis 4 an einem Filter.

7. Verwendung einer antiallergischen Zusammensetzung umfassend mindestens eine Pflanze oder einen Extrakt, ausgewählt aus der Oleaceae-Gruppe bestehend aus *Olea spp*. und *Ligustrum spp*., und ein hygroskopisches Material zur Denaturierung von Allergenen.

8. Verwendung einer antiallergischen Zusammensetzung umfassend Oleuropein und ein hygroskopisches Material zur Denaturierung von Allergenen.

9. Verwendung der antiallergischen Zusammensetzung zur Denaturierung von Allergenen gemäß Anspruch 7 oder 8, wobei das hygroskopische Material ein Erdalkalimetallsalz ist.

10. Antiallergische Zusammensetzung gemäß Anspruch 4, wobei das hygroskopische Material ein Chlorid oder Sulfat von Magnesium oder Calcium ist.

11. Antiallergische Zusammensetzung gemäß Anspruch 4, wobei das hygroskopische Material Calciumchlorid oder Magnesiumchlorid ist.

## Revendications

1. Composition anti-allergique qui comprend au moins un végétal ou un extrait sélectionné dans le groupe des oléacées constitué de *Olea spp*. et *Ligustrum spp*. et une matière hygroscopique, où la quantité de matière hygroscopique est de 0,5 à 25 % en poids de la composition.

2. Composition anti-allergique selon la revendication 1, qui comprend un extrait de *Olea spp.*

3. Composition anti-allergique qui comprend de l'oleuropéine et une matière hygroscopique.

4. Composition anti-allergique selon l'une quelconque des revendications 1 à 3, où la matière hygroscopique est un sel de métal alcalino-terreux.

5. Filtre ou feuille traité(e) par la composition anti-allergique selon l'une quelconque des revendications 1 à 4.

6. Procédé de dénaturation d'allergènes existant dans un environnement par application à un filtre de la composition anti-allergique selon l'une quelconque des revendications 1 à 4.

7. Utilisation d'une composition anti-allergique qui comprend au moins un végétal ou un extrait sélectionné dans le groupe des oléacées constitué de *Olea spp*. et *Ligustrum spp*. et une matière hygroscopique pour la dénaturation d'allergènes.

8. Utilisation d'une composition anti-allergique qui comprend de l'oleuropéine et une matière hygroscopique pour la dénaturation d'allergènes.

9. Utilisation de la composition anti-allergique pour la dénaturation d'allergènes selon la revendication 7 ou 8, où la matière hygroscopique est un sel de métal alcalino-terreux.

10. Composition anti-allergique selon la revendication 4, où la matière hygroscopique est un chlorure ou sulfate de magnésium ou de calcium.

11. Composition anti-allergique selon la revendication 4, où la matière hygroscopique est du chlorure de calcium ou du chlorure de magnésium.
